# EUROPEAN PATENT APPLICATION

(11) **EP 1 637 085 A2**
(43) Date of publication of application: **22.03.2006**
(21) Application number: 05017827.6
(22) Date of filing: 09.04.1996
(51) Int. Cl.: A61B 17/80

(54) **A polyaxial locking screw collar and plate assembly**

(30) Priority: 13.04.1995 US 421087
(62) Divisional of application: 03015953.7
(71) Applicant: Fastenetix, L.L.C., Summit, NJ 07901 (US)
(72) Inventor: Errico, Joseph P., Far Hills NJ 07931 (US); Errico, Thomas J., Summit NJ 07901 (US); Ralph, James, Oakland NJ 07436 (US); Tatar, Steve, Montville NJ 07045 (US)
(74) Representative: Gates, Marie Christina Esther

(57) **Abstract**

The present invention is a polyaxial locking screw plate assembly for immobilization of bones, via fixation thereto. In one embodiment, the invention includes a plate (100a) having axially tapered through holes (110a), into which holes bones screws (120), which have semi-spherical heads (122) rotationally freely mounted within the interior volumes of axially tapered coupling elements (132), may be inserted. The bone screws (120) may be inserted at a variety of angles which still permit the coupling elements (132) to seat within the holes (110a). The holes (110a) and the coupling elements (132) may be smooth, or threaded. The holes may be elongate so that during insertion the coupling elements (132) travel along the hole (110a), to provide dynamic compression to the bone. In a different embodiment, the holes (110b) may be partially threaded and partially tapered so that a two-piece coupling element may be utilised. In another embodiment, the coupling element may include an annular flange (180) portion.

## Description

### Background of the Invention:

### 1. Field of the Invention:

This invention relates generally to a orthopaedic implant assemblies for holding adjacent bones fixed. More particularly, this invention relates to a novel assembly of bone screws, collars, and plates for use in surgical procedures for stabilizing the relative motion of, or permanently immobilizing, bones, wherein the screws and collars form polyaxial couplings which are lockable in a receiving hole in a plate through a wide range of angulations.

### 2. Description of the Prior Art:

Human bones are constructed of a hard external shell, referred to as cortical bone, and an interior which is less dense and is referred to as cancellous bone. Although bones are generally resilient and structurally remarkable with respect to normal long term cyclical loading, extreme loading, a variety of traumas, and diseases may cause bones to fracture. Long bone fractures may be classified according to their severity, ranging from minor stress fractures to fully displaced fractures.

In cases of serious fractures of long bones, including those in which bone continuity is totally disrupted by a complete displacement of segments thereof, commensurately serious measures must be applied to correct the damage, and to permit a "callus" to form around the bone whereby union of the fracture is achieved. In many cases it is sufficient to "set" the bone by initially placing the surfaces of the bone segments which need to join in contact with one another, and applying rigid external immobilization. External casts and braces are utilized to provide stabilization of the bone, as well as to eliminate excessive stress loading during the healing process. Stress loadings of a light to moderate level are conducive to bone growth, however, if the stresses during healing are excessive, the fracture may not heal, and will alternatively go on to form a "non-union".

In the most severe of instances, however, the segments of the bone which is broken, and the manner in which the break manifests itself, makes the reduction of the fracture and the maintenance of the reduction difficult. In some cases the bone segments are displaced to the extent that invasive surgical procedures must be carried out in order to openly reduce the bone segments together. In others, the specific bone which is broken (such as the femur or the hip) is one for which the time involved with total healing exceeds what may practically be externally immobilized. In still others, the ability of a cast to sufficiently relieve the loadings associated with prudently constrained activity is insufficient to permit correct healing. In such cases it is often necessary to openly reduce and internally affix metal devices such as screws, rods, and plates to the bone segments.

An important class of orthopaedic conditions for which immobilization via internal fixation is frequently required concerns the spine. The spinal column of bones is highly complex in that it includes over twenty bones coupled to one another, housing and protecting critical elements of the nervous system having innumerable peripheral nerves and circulatory bodies in close proximity. In spite of these complications, the spine is a highly flexible structure, capable of a high degree of curvature and twist in nearly every direction.

Genetic or developmental irregularities, trauma, chronic stress, tumors, and disease are a few of the causes which can result in spinal pathologies for which permanent immobilization of multiple vertebrae may be necessary. A variety of systems have been disclosed in the art which achieve this immobilization by implanting artificial assemblies in or on the spinal column. The region of the back which needs to be immobilized, as well as the individual variations in anatomy, determine the appropriate surgical protocol and implantation assembly. The use of screw and plate assemblies for stabilization and immobilization via lateral or anterior entrance is common. Additionally, the use of plate assemblies in the posterior cervical spine is also common.

Because bones, once healed (or, in the case of immobilized sequences of vertebrae, fused) are routinely subject to high loads which cycle during movement, one of the primary concerns of physicians performing implantation surgeries, as well as of the patients in whom the implants are placed, is the risk of screw pull-out. Screw pull-out occurs when the cylindrical portion of the bone which surrounds the inserted screw fails. A bone screw which is implanted perpendicular to the plate is particularly weak because the region of the bone which must fail for pull-out to occur is only as large as the outer diameter of the screw threads. It has been found that for pull-out to occur for a pair of screws which are angled inward, "toe nailed", or ones which diverge within the bone, the amount of bone which must fail increases substantially as compared with pairs of screws which are implanted in parallel along the axis that the loading force is applied. It has, therefore, been a goal of those in the art to provide screw plate assemblies which permit screws to be entered into the bone at angles other than 90 degrees.

Another concern, especially in the spine, is that there are important internal tissue structures which, because of their proximity to the implant, may be damaged by a dislocated screw. In the cervical spine for example, the esophagus is located directly in front of the anterior surface of the vertebral body, and therefore, in potential contact with an implanted anterior cervical plate. Breaches of the esophageal wall permit bacterial contamination of the surrounding tissues, including the critical nerves in and around the spinal cord. Such contamination can be fatal.

Because screw pull-out represents one of the largest risks of implant failure, it has been an object of those in the art to produce a screw and plate designs having a locking means which couples, not only the plate to the bone, but locks the screw to the plate. In such a design, it is intended that, even if the bone holding the screw fails, the screw will not separate from the plate.

With respect to the reduction of long bone fractures, a very successful advance over standard screw plate assemblies are dynamic compression plates (DCPs). In its most widely used form, the DCP is an elongate planar metal plate having a plurality of sequentially spaced elongated holes, the holes being coaxial and extending along the axis of the plate. Each hole, which extends fully through the plate, is defined by a pair of vertically oriented parallel sidewalls. The top of the parallel sidewalls, near the top surface of the plate, includes an additional recessed lip on which the annularly extending portion of the head of a screw rests once inserted into the hole. The top of the lip is, therefore, lower than the top surface of the plate. The elongate profile of the lip defines a downward slope with respect to the top surface of the plate. wherein the distance between the top of the lip surface and the top surface of the plate increases along the elongate axis of the hole. The downward slope of the lip of each hole is oriented toward the middle of the plate.

In use, the plate is positioned next to the exposed surface of the broken, and potentially displaced, bone such that one portion, an upper portion of the plate is adjacent to one of the segments, and the holes of the lower portion, are adjacent to the other bone segment. First and second screws are inserted through the plate into opposing segments of the broken bone, at respective points along the axial extent of the elongate holes. It is preferred that the respective insertion points be at least some distance from the end of the hole having the lip at its greatest depth, herein referred to as the proximal end. In general, the screws are generally inserted at the uppermost end of the elongate holes, herein referred to as the remote end.

Once inserted, the screws are driven into the bone, therein securing the plate to the respective bone segments. The compressing function of the plate is utilized by continued driving of the screw into the plate once the annularly extending portion of the screw head has seated against the recessed lip of the hole. The downward force applied to the screw causes the head to slide down the slope of the recessed lip, thereby causing the bone in which it is inserted to move relative to the fixed plate. As the slopes of the respective holes are mutually directed toward the middle of the plate, the relative motion of the screws down the sloped lips, and the corresponding motion of the bone segments into which they have been inserted, is together. The surgeon is thereby able to draw displaced bones together, and to hold the bones together internally during the required healing time.

Unfortunately, however, dynamic compression plates suffer the same failure mechanisms as other screw and plate devices, e.g., screw pull-out. While variations of the DCP concept, specifically those which include additional holding screws for anchoring the plate once compression has been applied, have reduced the dangers associated with bone screw pull-out with such plates, the risks associated with this failure mechanism have not been eliminated. Other DCP systems which have provided for angulation of screws have generally been limited to single angles. Surgeons ideally need to have freedom with respect to insertion angles so that the individual variations in fracture type, and bone anatomy may be accounted for in the fixation means. At the same time, however, once implanted, the screws should not be subject to pull-out.

The present invention is, therefore, directed to providing a new and novel screw and plate design having a polyaxial coupling of the screw to the plate, whereby a single plate is compatible with a wide range of screw-in angles.

Further, the present invention provides a simple and effective locking mechanism for locking the bone screw to the plate.

It is additionally a principal feature of embodiments of the present invention to provide a new and novel dynamic compression plate having a polyaxial coupling of the screw to the plate.

Another principal feature of aspects of the present invention is the ability to provide a dynamic compression screw plate having a locking compression screw and hole.

Other features and advances of the present invention not explicitly stated will be set forth and will be more clearly understood in conjunction with the descriptions of the preferred embodiments disclosed hereafter.

### Summary of the Invention:

The preceding objects of the invention are achieved by the present invention which is a locking polyaxial screw plate assembly for use in stabilizing and immobilizing bones. In its various embodiments, the invention comprises a plate having a plurality of holes, bone screws having semi-spherical heads, and coupling elements. The coupling elements may comprise a variety of different embodiments, however, each includes an interior volume shaped to receive and polyaxially retain the semi-spherical head therein, and an exterior conformation for insertion and locking into the holes of the plate. The coupling elements each include a slot and/or circumferential discontinuity such that the radius of the element may be expand or contract by the application of a radial force whereby the interior conformation may compress against the semi-spherical head top portion of the bone screw once the coupling element is inserted and locked in the hole, thereby locking the screw in the desired angle.

In its first embodiment, which is an anterior spinal implant having a unitary coupling element, the plate is a flat metal element, having rounded corners, contoured to the curved cylindrical surface of the vertebral bodies to which it is secured. There are four tapered holes disposed in pairs at the opposing ends of the plate. These holes may be threaded or smooth (corresponding to the type of coupling element being utilized in conjunction therewith), and are positioned so that they are aligned in pairs with the vertebral bodies to which the plate is to be attached.

The threading and shaft portion of the bone screws may be of a variety of standard designs, or a particular design which may be found more secure than the standard ones. The head, however, is not standard in that it comprises a semi-spherical section. For the purposes of inserting the screw into the bone, the head comprises a recessed region such as a slot, phillips, star, or hexagonal recesses which are ideally suited for mating to an appropriate screwdriving tool. The recess, however, does not alter the exterior radially symmetric, semi-spherical shape, of the head.

The coupling element comprises an interior volume which holds the ball head of the screw, and an exterior surface taper which conforms to the taper of the corresponding hole. The coupling element may be threaded or smooth, but in either instance the coupling element includes at least one vertical slot such that the coupling element may expand and contract to receive and/or lock the head of the screw in the interior volume. The size of the tapered hole, relative to the coupling element, is such that the full insertion of the element into the hole causes the slots to contract and the interior surface of the coupling element to lock to the surface of the screw head.

In the variation of this anterior spinal plate in which the coupling element and the tapered holes are smooth, it is understood that the final step of inserting the screw into the bone causes the coupling element to seat in the hole and to be drawn down into the hole, thereby locking the coupling element to the screw, the screw at a desired angle relative to the plate, and the coupling element within the hole. In the variation of this embodiment, suggested above, in which the coupling element and the tapered holes include corresponding threadings, it is understood that the final step of the insertion of the screw and coupling element into the holes further includes a rotation of the coupling element into the hole. This is naturally occurring in a device wherein the threading of the collar is of the same helical orientation as the screw shaft. In the alternative, a separate screwdriving tool may be utilized to insert the coupling element into the plate. In this case, as above, the act of locking the coupling element in the hole causes the interior surface of the coupling element to crush lock to the surface of the head of the screw, thereby locking the screw at its proper angulation relative to the plate. Each of these variations may further include retaining rings integrally formed within the upper portions of the holes. Each retaining ring expands as the corresponding coupling element is inserted into the hole, and snaps back into a retaining position above the coupling element once the top of the element passes it into the hole.

In both variations, the top of the coupling element provides an opening which may be positioned co-linearly with the recess in the screw head for driving in the screw. This opening is desirably large enough such that the screw and coupling element may be manipulated easily and the head of the screw is accessible even at entrance angles which are far from perpendicular.

The first step in the process of implanting these types of anterior spinal implants is to position the plate against the vertebral bodies and to align the entry points for the screws. The next step is to pre-drill the holes into the bones at the desired angle, into which the screws will be inserted. With the plate in place, the screws may now be inserted through the holes in the vertebral bodies. Once the screw has been substantially fully inserted into the bone, at the desired angle, the coupling element contacts the top of hole in the plate. If the coupling element is not aligned properly with the hole, it rotates via its polyaxial mounting to the screw to seat into the hole so that it may be locked down into the plate (either by continued driving of the screw into the bone, or, in the case of the threaded coupling element, by separate rotation of the coupling element).

An alternative embodiment for use in the anterior spine, includes a similar plate having a set of holes for receiving a screw and coupling element therethrough. In this embodiment, however, the holes are untapered and threaded through the top portion thereof, and tapered and smooth at the bottom. In addition, the coupling elements are not unitary elements, but are rather two-piece elements. The top piece of the coupling element has an external threading for locking with the threaded end of the holes in the plate, and also has an interlocking mechanism for joining with the lower piece of the coupling element. The lower piece of the coupling element has a semi-spherical interior volume, which is expandable and contractable, for receiving the semi-spherical head of the screw therein. The bottom portion of the lower piece of the coupling element further includes an exterior taper which mates with the tapered bottom of the corresponding hole in the plate, so that there is an inwardly directed force applied to the lower piece, thereby causing the interior volume of the coupling element to contract and lock to the head of the screw.

More particularly, in this two-piece coupling element embodiment, the two part coupling element comprises and socket portion and a cap portion. The socket portion is designed with an interior semi-spherical volume, so that it may receive the semi-spherical head of a corresponding bone screw. The interior volume of the socket portion is open at both axial ends thereof. The exterior surface of the socket portion, at the bottom thereof, includes a first set of slots which extend upwardly from the opening so that the interior semi-spherical volume may be expanded or contracted by the application of a radial force. In addition, the exterior surface at the bottom is tapered so that it is narrower at the bottom than at a midpoint.

The upper exterior surface of the socket portion comprises a second set of slots, directed axially along the element to the midpoint, such that the upper opening of the socket element may expand and contract in accordance with the application of a radial force thereon. The exterior surface of this upper section of the socket portion is not tapered and is narrower than the widest taper position of the bottom of the socket portion. The upper section, however, does further include an outwardly extending annular lip at the uppermost axial position. This upper section is designed to be inserted into, and joined with, the cap portion of the coupling element.

The cap portion has a generally cylindrical shape, having an open bottom. The open bottom is inwardly tapered, forming an inwardly extending annular lip, so that as the upper end of the socket portion is inserted, its upper slots are narrowed. Once axially inserted beyond this taper, the upper section of the socket portion expands outward over the inwardly extending annular lip. The inwardly extending annular lip engages the outwardly extending lip of the socket portion so as to prevent disengagement of the two pieces. The socket portion is then permitted to slide into the cap portion, until the larger diameter of the tapered lower portion of the socket contacts the entrance of the cap portion.

The exterior surface of the cap portion is threaded, so that it may engage the threading of the upper portion of the corresponding hole. In addition, the top of the cap includes an opening so that a screw driving tool may directly engage the top of the screw.

The first step in the process of implanting this embodiment of the invention is to assemble the parts described above. (It is intended that this assembly occur at the manufacturing site, and not be the responsibility of the surgical staff.) The semi-spherical head of the screw is inserted into the interior volume of the socket portion, and held in place by the interference fit of the maximum diameter of the head with the unexpanded openings at the top and bottom of the element. The head is inserted from the bottom, and more specifically by applying a pressure which causes the bottom opening to expand to receive the head into the volume.

Once the screw head is inserted, the upper end of the socket portion is inserted into the opening of the cap portion. This insertion causes the top of the socket portion to contract inward slightly until the annular lips of each portion engage one another. The socket portion and the cap portion are thereby joined loosely so that each may slide and rotate relative to one another.

The surgeon then positions the plate against the vertebral bodies and aligns the entry points for the screws. The next step is to pre-drill the holes into the bones at the desired angle, into which the screws will be inserted. With the plate in place, the screws (with the corresponding socket and cap portions in place on the head of each screw) are inserted through the holes of the plate, and into the vertebral bodies. The coupling element provides axial access to the screw head for driving it into the bone. At all times during this insertion, the head of the screw is loosely retained within the coupling element, so that the coupling element may polyaxially angulate relative to the screw, within a range of angles defmed by the diameter of the neck of the screw and the bottom opening of the socket portion.

As the screw is inserted into the bone, at the desired angle, the socket portion of the coupling element angulates so that the tapered bottom portion thereof seats into the tapered bottom of the hole in the plate. Continued driving of the screw into the bone, and therefore the socket portion of the coupling element deeper into the tapered hole, causes the first set of slots in the bottom end of the socket portion to narrow, thus causing the head of the screw to be crush locked to the coupling element. The cap portion of the coupling element is then threadably inserted into the hole, locking the coupling element in the hole, and further driving the socket portion into the hole.

In a preferred variation of this embodiment, the interior surface of the cap portion includes a slight narrowing taper so that as the cap is threaded downward into the hole in the plate, the upper slots of the socket portion are also narrowed, further increasing the crush locking effect on the head of the screw.

It shall be understood that the shape of the plate, and the distribution of the holes thereon may be altered to correspond to other areas of the spine, for example the posterior cervical region wherein the plate may be aligned against and coupled to the lateral masses. Further, as a standard bone plate, the polyaxial locking screw/coupling element/plate features described in the preceeding embodiments may be applied as general bone plates.

An advanced form of such a general bone plate embodiment, however, requires additional description inasmuch as it provides dynamic compression. More particularly, the dynamic compression plate aspect of the present invention has several alternative embodiments, two of which are introduced hereinbelow. Each embodiment includes an elongate flat metal plate element which is contoured to the curved cylindrical surface of the specific bone to which it is to be secured. In addition, it is desirable that the plate also comprise a plurality of round holes for receiving therethrough additional holding screws which may be polyaxial screws and coupling elements as are set forth above.

In each dynamic compression embodiment, the coupling element of this embodiment comprises a socket for holding the ball head of the screw, and an external conformation which permits the screw and coupling element to lockably mate with the sidewall morphology of the corresponding elongate hole.

More specifically with respect to the sidewall morphology, in a first embodiment, the at least one elongate hole (preferably a pair of holes) extends in the direction of the elongate axis of the plate. The sidewalls of the elongate hole of this embodiment are tapered in two axes; the first axis being perpendicular to the plane of the plate, such that the hole is narrower at the bottom than at the top, and the second axis being coaxial with the hole itself, such that the hole is wider at the end closest to the middle of the plate (as measured with respect to the longitudinal axis of the plate), the proximal end.

The coupling element which corresponds to this first embodiment, therefore, comprises a cylindrical element, tapered to form a frusto-conical section. It is effectively identical to the coupling element of the first embodiment of the anterior spinal screw plate assembly introduced above (with the smooth exterior surface). The width of the top of the elongate hole, at the remote end thereof which is farthest from the middle of the plate, the remote end, is slightly wider than the diameter of the bottom of the coupling element, and is considerably narrower than the top of the coupling element. The tapered sidewalls of the plate widen in the direction of the middle of the plate, toward the proximal end wherein the width of the top of the elongate hole at the proximal end, is equal to the diameter of the top of the coupling element. The width at the bottom of the elongate hole widens as well, in the direction of the middle of the plate, however, at the proximal end, the width of the bottom of the hole widens at a slower rate than the top of the hole. It is this slight difference in tapering which compression locks the coupling element to the screw head and to the plate.

The first step in the process of implanting this embodiment of the invention is to position the plate against the segments of the broken bone which require compression and immobilization, and to align the entry points for the screws. The next step is to pre-drill holes into the bones at the desired position along the elongate hole, and angle with respect to the plate, into which the screws will be inserted. With the plate in position, the first and second screws are inserted through the elongate holes into the bone segments.

Once the screw has been inserted into the bone, at the desired angle, the coupling element, via its rotationally free mating to the socket in the inserted screw, is realigned so that it seats in the elongated hole, the tapered surface of the coupling element fitted to the tapered walls of the hole. Continued rotation of the screw causes the coupling element to slide deeper along the walls of the hole, which correspondingly causes the screw and bone to move relative to the plate. The coupling element continues to slide down the ever widening tapered walls of the hole, until it reaches the proximal end. At the proximal end, the slightly narrowed bottom of the hole crush locks the coupling element to the screw head, therein locking the screw to the coupling element, as well as compression locking the coupling element to the plate.

In the second embodiment of the dynamic compression plate, the elongate holes are not tapered along the axis which is perpendicular to the plane of the plate, but rather each sidewall comprises a sloped channel. This elongate hole does, however, retain a slight taper at the proximal end of the hole. At the remote end, the channel comprises only a recessed lower lip, the lip forming the upper extent of the lateral channel which is sloped downward into the plate. Once the channel has extended a distance large enough to provide an initial seating of the coupling element onto the lower lip, an upper lip portion, which defines the upper extent of the channel, is introduced.

The coupling element of this embodiment comprises an annular flange which seats on the lower lip of the hole in an initial position, and is driven by continued insertion of the screw down the slope of the channel. Once the coupling element begins to translate down the channel, the upper lip extends over the annular flange and prevents the coupling element from pulling out. As above, this coupling element includes at least one vertical slot, either extending downwardly from the top (such that the element compresses onto the head of the screw from above), upwardly from the bottom (such that the element compresses onto the head of the screw from below), or completely from the top to the bottom of the device (a single slot thereby rendering the coupling element as a 7/8ths ring, and such that the element compresses onto the head entirely circumferentially),

Implantation of this apparatus is initiated in a manner which is generally similar to the first embodiment: the plate and holes are aligned, the pre-drill holes are provided at the appropriate angulations, the screws and their corresponding coupling elements are driven through the elongate holes and into the bone. Once the screw is inserted, the annular flange of the coupling element seats on the lower lip of the channel and begins to translate down the slope in accordance with continued driving of the screw into the bone. The bone and screw move relative to the plate, both moving closer to the middle of the plate. As the annular flange of the coupling element travels, it becomes vertically locked beneath the upper lip of the channel. The tapering of the hole in the axial direction, at the proximal end, causes a radial compression of the coupling element, therein locking the ball head to the coupling element, in the same manner as the first embodiment (i.e., slots in the coupling element), but locking the screw to the plate via the annular flange and the channel.

### Brief Description of the Drawings:

Figures 1a and 1b are perspective views of locking plates which are aspects of the embodiments of the present invention.
Figure 2 is a perspective view of a bone screw which is an element of one aspect of the present invention.
Figures 3a and 3b are side views of coupling elements which are aspects of embodiments of the present invention.
Figures 4a and 4b are side cross-sectional views of the fully assembled embodiments of the present invention, the elements of which are shown in Figures 1a, 2, and 3a, and Figures 1b, 2, and 3b, respectively.
Figure 5 is a top view of an alternative plate of the present invention further including integral retaining rings formed in the upper portion of the holes.
Figure 6 is a side cross-section view of the fully assembled form of the embodiment of the present invention including the plate as shown in Figure 5.
Figure 7 is a side cross sectional view of a hole in the plate of another embodiment of the present invention.
Figures 8a and 8b are side cross sectional views of cap and socket portions of a two-piece coupling element of another embodiment of the present invention.
Figure 9 is a side cross section view of a fully assembled form of the embodiment of the present invention which includes the elements illustrated in Figures 2, 7, and 8a-b.
Figure 10 is a side cross section view of a fully assembled form of a variation of the embodiment of the present invention shown in Figure 9, wherein the inner surface of the cap portion is tapered.
Figure 11 is a side view of a dynamic compression plate of the present invention.
Figure 12 is an axial side view of an an elongate hole of an embodiment of the dynamic compression plate aspect of the present invention.
Figure 13 is a side cross-section view of an assembled dynamic compression plate which includes the elements shown in Figures 2, 3a, 11 and 12.
Figure 14 is a side view of a coupling element for use with an alternative dynamic compression plate.
Figures 15a and 15b are side cross-section views, taken along mutually perpendicular axes, of an elongate hole of another embodiment of a dynamic compression plate of the present invention.
Figure 16 is a side cross-section view of the fully assembled dynamic compression plate embodiment, the elements of which are shown in Figures 2, 14, and 15a-b.

### Detailed Description of the Preferred Embodiment:

While the present invention will be described more fully hereinafter with reference to the accompanying drawings, in which particular embodiments and methods of implantation are shown, it is to be understood at the outset that persons skilled in the art may modify the invention herein described while achieving the functions and results of this invention. Accordingly, the descriptions which follow are to be understood as illustrative and exemplary of specific structures, aspects and features within the broad scope of the present invention and not as limiting of such broad scope. Like numbers refer to similar features of like elements throughout.

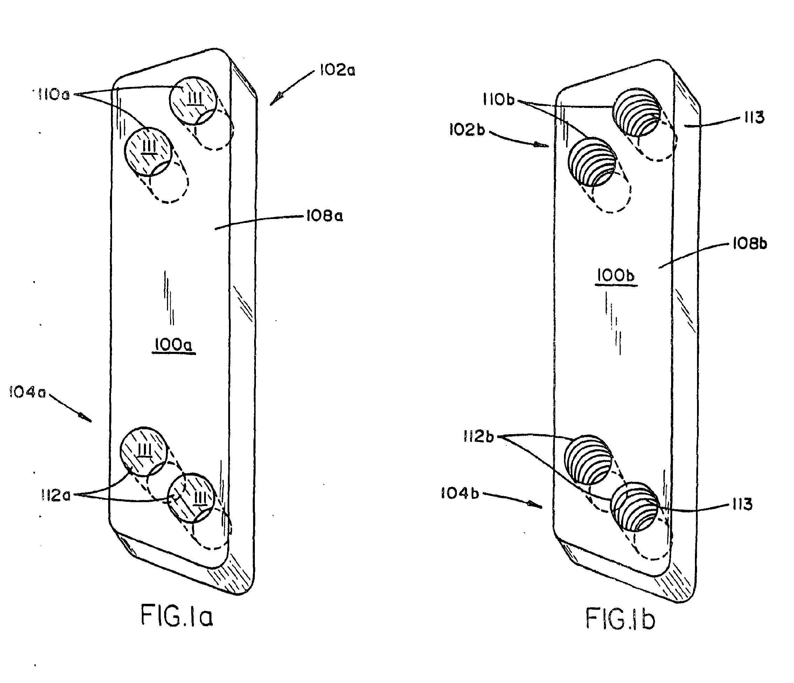

Referring now to Figure 1a, a plate which is an element of the present invention is shown in a perspective view. The plate 100a may be constructed of any suitably biocompatible material which has the structural strength and durability to withstand the cyclical loading associated with long term fixation to the spine. Materials which would be suitable for such applications include titanium alloys and steels. A specific titanium material which has been utilized in implants of the prior art include ASTM F-136 titanium alloy (Ti 6AL-4V). This material has enhanced mechanical properties including fatigue endurance and tensile strength, as compared with pure titanium.

The plate 100a comprises upper and lower portions 102a,104a respectively, and a top surface 108a and a bottom surface (not shown). A slight curvature is generally imparted to the plate 100a so that it may grossly conform to the cylindrical morphology of the bones (i.e., vertebral bodies) which it couples. The curvature is correspondingly such that the top surface 108a is the convex surface, the bottom surface is concave.

A pair of holes 110a, having a smooth tapered inner surface 111. extend fully through the upper portion 102a of the plate. A second pair of holes 112a having a tapered inner surface 111 as well, are disposed in the lower portion 104a of the plate 100a. Each of the holes 110a, 112a is ideally suited for receiving therethrough a bone screw for affixing the plate to the vertebral bodies.

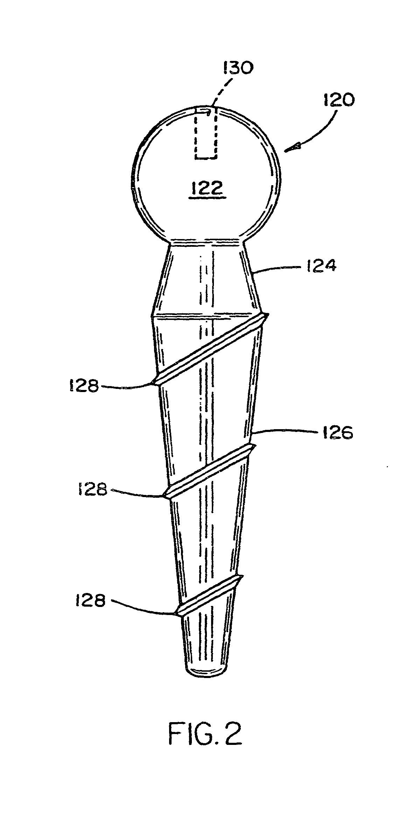

Referring now to Figure 2, a screw of a type which is ideally suited for coupling the plates of this invention to vertebral bodies (or long bones in other embodiments) is shown in a side view. The screw 120 comprises a head portion 122, a neck 124, and a shaft 126. In Figure 2, the shaft 126 is shown as having a tapered shape with a high pitch thread 128. It shall be understood that a variety of shaft designs are interchangeable with the present design. The specific choice of shaft features, such as thread pitch, or shaft diameter to thread diameter ratio, or overall shaft shape, etc. should be made be the physician with respect to the conditions of the patient's bone, however, this invention is compatible with a wide variety of shaft designs.

The head portion 122 of the screw 120 comprises a semi-spherical shape, which has a recess 130 in it. It is understood that the semi-spherical shape is necessarily is a section of a sphere, greater in extent than a hemisphere, and exhibits an external contour which is equidistant from a center point of the head. In a preferred embodiment the major cross-section of the semi-spherical head 122 (as shown in the two dimensional illustration of Figure 2) includes at least 270 degrees of a circle.

The recess 130 defines a receiving locus for the application of a torque for driving the screw 120 into the bone. The specific shape of the recess 122 may be chosen to cooperate with any suitable screwdriving tool. For example, the recess 130 may comprise a slot for a flat-headed screwdriver, a crossed recess for a phillips head screwdriver, a hexagonally shaped hole for receiving an allen wrench, or most preferably, a threading for receiving a correspondingly threaded post. It is further preferable that the recess 130 be coaxial with the general elongate axis of the screw 120, and most particularly with respect to the shaft 126. Having the axes of the recess 130 and the shaft 126 colinear facilitates the step of inserting the screw 120 into the bone.

The semi-spherical head portion 122 is connected to the shaft 126 at a neck portion 124. While it is preferable that the diameter of the shaft 126 be less than the radius of the semi-spherical head 122, it is also preferable that the neck 124 of the screw 120 be narrower than the widest portion of the shaft 126. This preferable dimension permits the screw to be inserted at a variety of angles while still permitting the specific coupling element to be screwed into the appropriate hole 110a or 112a of the plate 100a and remain coupled to the head 122.

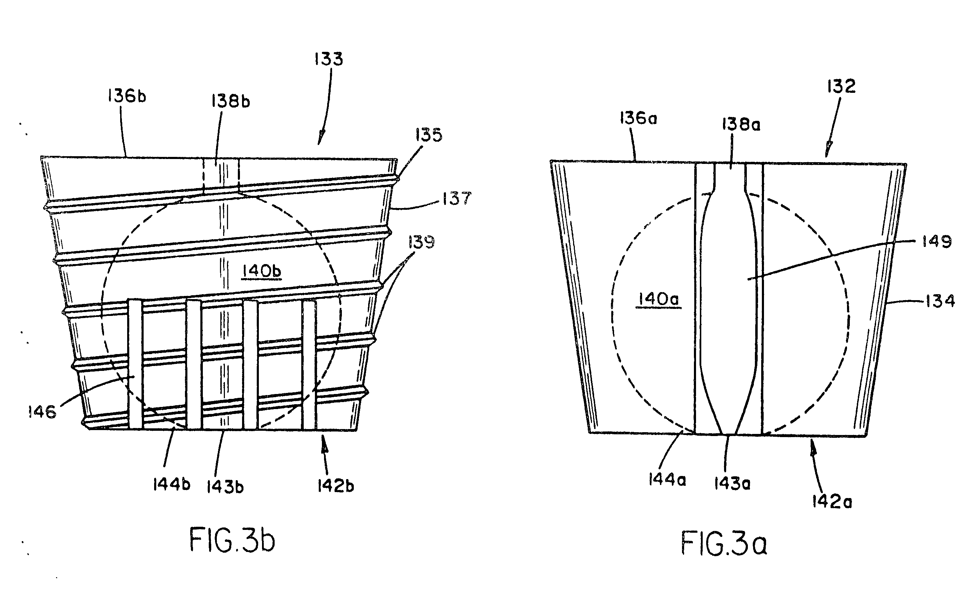

Referring now also to Figure 3a the first variation of the coupling element 132, which is utilized in conjunction with the plate 100a shown in Figure 1a, is shown in side views, wherein phantom lines show the interior structure of the elements along a diametrical cross section. The coupling element 132 comprises a tapered cylindrical socket having a smooth external surface 134. The taper of the surface 134 is designed to mate with the interior tapered surface of the holes 110a or 112a of the plate 100a, so that the coupling element 132 may be seated into the plate 100a.

The top surface 136a of the coupling element 132 further comprises a through hole 138a, which extends from the top surface 136a to an interior semi-spherical volume 140a. This through hole 138a is designed such that the screwdriving tool which is used to insert the screw 120 into the bone may access and rotate the screw 120 through the coupling element.

The coupling element 132 further includes an interior semi-spherical volume 140a which is ideally suited for holding the head portion 122 of the screw 120, and permitting the screw to rotate through a range of angles. The bottom 142a of the coupling element 132 has a circular hole (enumerated as 143a on the bottom surface of the side view of the coupling element) which forms the bottom entrance into the interior semi-spherical volume 140a. It is understood that the head 122 of the screw 120 is held within the interior semi-spherical volume 140a by the annular rim, or support lip, 144a of the bottom 142a of the coupling element. This annular support lip 144a defines the circular opening 143a which has a diameter less than the diameter of the semi-spherical head 122 of the screw 120, but larger than the neck 124 of the screw 120 (so that the screw 120 may polyaxially rotate relative to the coupling element 132 once the head 122 has been inserted into the interior volume 140a.

In the variation of this embodiment which is shown in Figure 3a the coupling element 132 comprises a single axial slot 149 so that the element 132 has a circumferentially discontinuous conformation. This renders the coupling element 132 a 7/8ths ring, and permits the element 132 to expand to accept the inserted head portion 122, and also to crush against the head 122 upon the application of an inwardly directed radial force. Insertion of the coupling elements into the through holes prevents the coupling elements from releasing the head 122 by preventing the circular opening 143a from expanding.

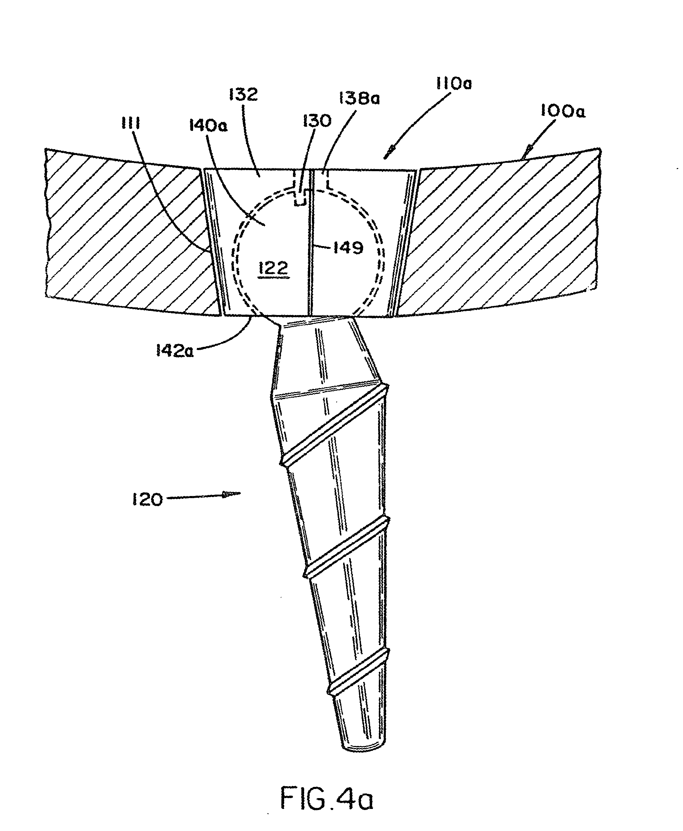

Referring now to Figure 4a. a cross sectional view of a fully assembled embodiment of the screw plate assembly of the present invention, including the smooth unitary coupling element 132 and the plate 100a having smoothly tapered holes 110a,112a, is shown. With reference to the relative positions of the screw 120, plate 100a, and coupling element 132, the operative steps of implanting this screw plate assembly and affixing it to a pair of vertebral bones begins with preparing the bones through surgical tissue exposure and/or resection. Next the plate 100a is positioned against the bones and pre-drill holes are made thereinto at the desired insertion angle for the screws 120. Each screw 120 and corresponding coupling element 132 are then joined together so that the head 122 is within the interior volume 140a, whereby the two elements are able to rotate freely with respect to one another, but are nonetheless coupled.

The recess 130 in the screw 120 and the through hole 138a of the coupling element 132 are aligned at first, and an appropriate screwdriving tool is used to insert the screw 120 through the proper hole 110a or 112a and into the pre-drilled hole in the bone. Once the screw 120 has been screwed down to the point that the bottom surface 142a of the coupling element contacts the top of the hole 1 10a or 12a, the coupling element 132 angulates into a proper seating position. Continued insertion of the screw 120 causes the tapered surfaces 134 and I I 1 of the coupling element 132 and the holes I 10a,112a, respectively, to engage. The taper 111 of the corresponding hole 110a or 112a causes the slot 149 to narrow, and the interior volume 140a of the coupling element to crush onto the surface of the head 122. Complete insertion of the coupling element 132 to the plate 100a, locks the coupling element and screw to the plate, as well as locking the screw 120 and plate 100a to the bone.

Referring now to Figures 1b, 2, 3b, and 4b, an alternative variation of the coupling element of this embodiment is shown in a side view, wherein the interior surfaces 113 of the holes 110b and 112b of the plate, as well as the exterior surfaces 137 of the corresponding coupling elements 133 are threaded.

More specifically with respect to Figure 3b, the threaded coupling element 133 is shown in a side view. This threading 139 is designed to mate with threading 113 of the holes 110b,112b of the plate 100b, so that the coupling element 133 may be screwed into the plate 100b. It is preferable that the uppermost thread 135 be designed to crush-lock so that the coupling element 133 is secure in the hole 110b.

In order for the opening 143b in the bottom 142b of the coupling element 133 to expand, thereby permitting the head 122 of the screw 120 to enter into the interior volume 140b, this variation of coupling element 133 includes a plurality of slots 146. These slots 146, as shown, extend upwardly from the bottom 142b to a point along the axial extent of the element 133 beyond the maximum diameter of the interior volume 140b. Threaded insertion of the coupling element 133 into the corresponding through hole 110b,112b causes the slots to narrow, thereby preventing the coupling element 133 from releasing the head 122 by preventing the circular opening 143b from expanding. The holes 110b,112b of the plate 100b are tapered inward such that complete insertion of the coupling element 133 thereinto causes the slots 146 to be compressed and, correspondingly, for the bottom entrance 143b and the corresponding annular lip 144b to crush lock the screw head 122 into position.

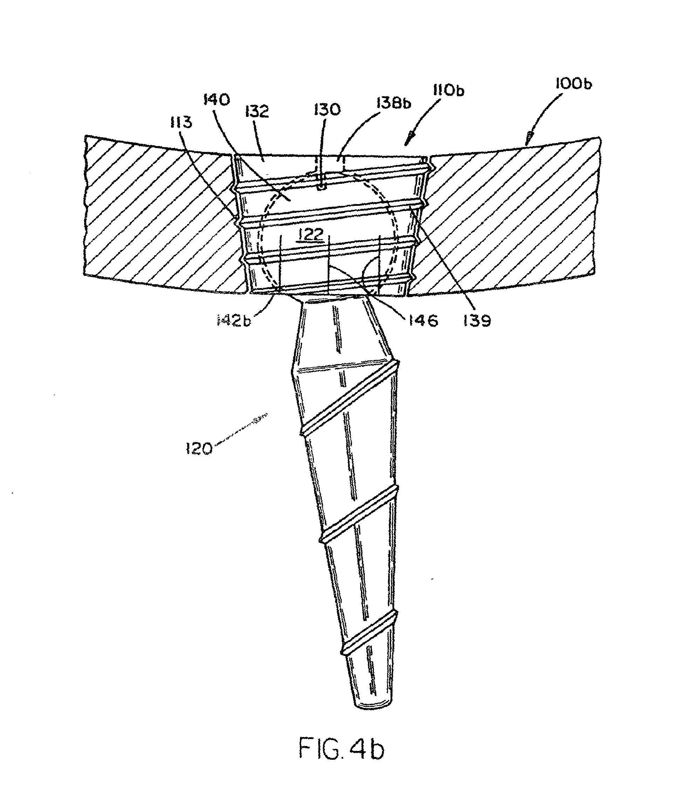

Referring now to Figure 4b, a cross sectional view of a fully assembled screw plate assembly of the present invention is shown. With reference to the relative positions of the screw 120, plate 100b, and coupling element 133, the operative steps of implanting this screw plate assembly and affixing it to a pair of vertebral bones begins with preparing the bones through surgical tissue exposure and/or resection. Next the plate 100b is positioned against the bones and pre-drill holes are made into the lateral masses at the desired insertion angle for the screws 120. Each screw 120 and corresponding coupling element 133 are then placed together so that the head 122 is within the interior volume 140b, whereby the two elements are able to rotate freely with respect to one another, but are nonetheless coupled.

The recess 130 in the screw 120 and the through hole 138b of the coupling element 133 are aligned at first, and an appropriate screwdriving tool is used to insert the screw 120 through the proper hole 110b or 112b and into the pre-drilled hole in the bone. Once the screw 120 has been screwed down to the point that the bottom surface of the coupling element 133 contacts the plate 100b, the first threads 139 of the coupling element are mated to the threading 113 of the hole 110b or 112b. Complete insertion of the coupling element 133 to the plate 100b, locks the coupling element and screw to the plate. as well as locking the screw 120 and plate 100b to the bone.

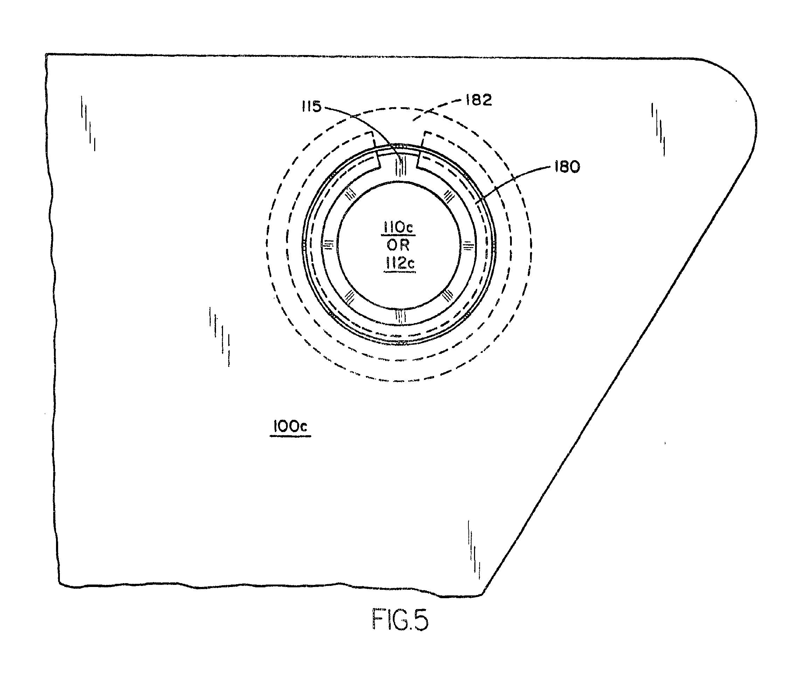

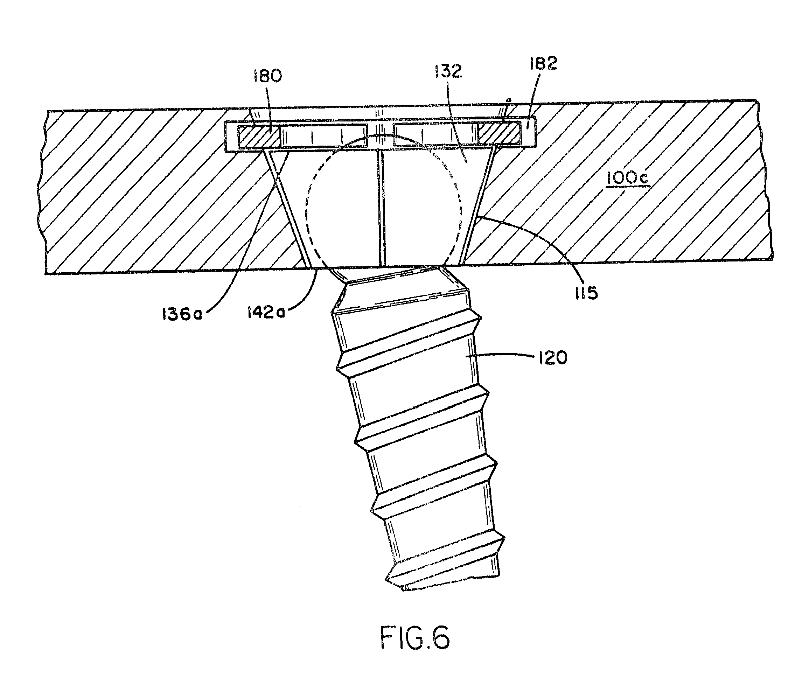

Referring now to Figures 5 and 6, a variation of the embodiments set forth above, in which there is a retaining ring provided to further lock the screw and coupling element within the tapered hole. More specifically with respect to Figure 5, which is a top view of the hole and retaining ring of this variation, the tapered hole 110c or 112c includes an annular recess formed 182 in the side wall 115 of the hole (the hole may be either smooth as in Figure I a or threaded as in Figure 1b). Within this annular recess 182 is provided a discontinuous "7/8ths" snap-ring 180. The undeflected outer diameter of the snap-ring is greater than the diameter of the hole 110c or 112c (so that it will remain in the recess), and is also less than the diameter of the top 136a or 136b of the coupling element 132 or 133, respectively. The inner diameter of the undeflected snap-ring 180 is, however, less than the diameter of the tapered hole, but is greater than the bottom 142a or 142b of the coupling element.

Referring now also to Figure 6, the assembled screw 120, coupling element 132. snap-ring 180 and plate 100c are shown. The snap-ring 180 is deflected inward for positioning in the recess 182. During the implantation procedure, the screw 120 and the coupling element 132 (or 133) are inserted through the hole. Once the bottom 142a (or 142b) of the coupling element 132 (or 133), respectively, seats in the top of the hole 110c or 112c, and begins to travel into it, the tapered exterior surface of the coupling element 132 (or 133) causes the snap-ring 180 to expand into the recess 182. Once the coupling element 132 (or 133) is fully seated in the hole 110c, the snap-ring 180 is freed from the outward radial pressure of the coupling element 132 (or 133) and snaps back to its undeflected state. In its undeflected state it prevents the coupling element 132 (or 133) from backing out of the plate 100c inasmuch as the flat upper surface 136a (or 136b) of the coupling element is incapable of deflecting the ring outward (it has no taper to push the snap-ring open).

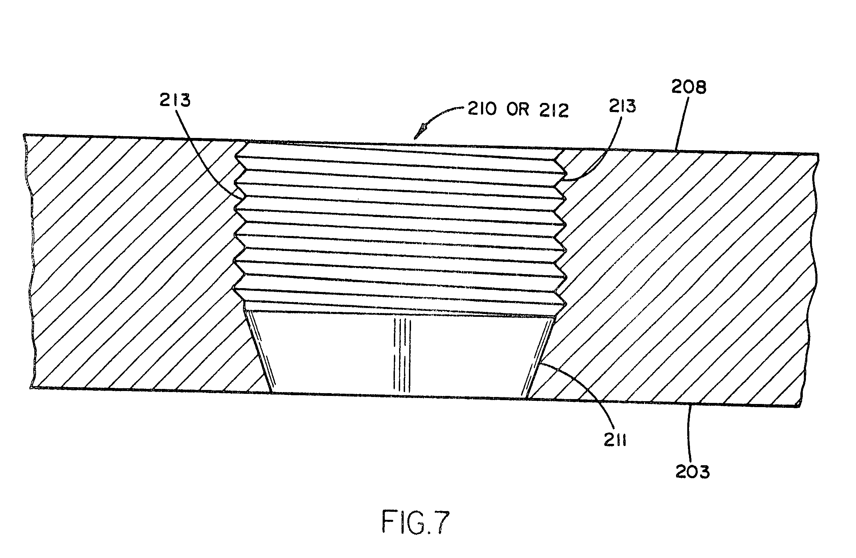

Referring now to Figure 7, a cross-section view of the hole 210 or 212 in plate 200, which otherwise similar to the plates 100a and 100b shown in Figures 1a and 1b, is shown. Extending from the top surface 208 of the plate, into the hole 210 or 212, to a position which is above the bottom surface 203 is a threading 213. The diameter of the hole 210 or 212 does not vary in this threaded region 213, however, the bottom section of the hole includes an inward taper 211 and is smooth.

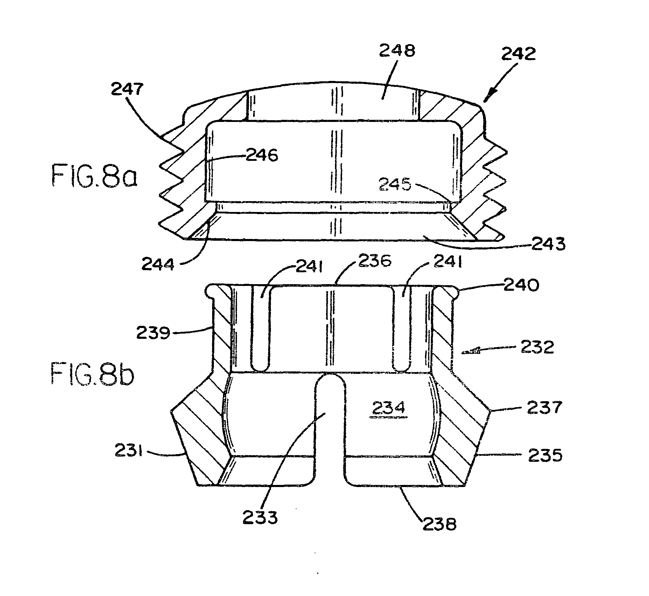

Referring now to Figures 8a and 8b, the two portions which form the coupling element of the present invention are shown in side cross-section views. Phantom lines show the interior structure of the elements along the diametrical cross section. With specific reference to the socket portion 232, shown in Figure 8b, the coupling element comprises a roughly cylindrical shape having an interior volume 234 in which the semi-spherical head 122 of the screw 120 is disposed. The interior volume 234 is open at the top 236 of the socket portion 232 and at the bottom thereof 238. The lower section 231 of the socket portion 232 comprises a set of slots 233 which extend vertically from the bottom 238 of the socket portion 232 to a position above the maximum diameter of the semi-spherical interior volume 234. These slots 233 permit the interior volume to expand and contract in accordance with the application of a radial force thereon. The external surface 235 of the lower section 231 of the socket portion 232 is tapered such that the narrowest part of the lower section 231 is at the bottom 238.

The upper section 239 of the socket portion 232, shown in Figure 8b, has a generally constant diameter, which is less than the diameter at the uppermost position 237 of the taper of the lower section 231. A second set of vertical slots 241 are provided in this upper section 239 so that it may also expand and contract in accordance with radial forces applied thereto. In addition, the uppermost end of this upper section 239 comprises an outwardly extending annular lip 240.

The cap portion 242 of the coupling element, shown in Figure 8a, comprises an opening 243 in the bottom thereof, having an inwardly tapered entrance surface conformation 244. As the upper section 239 of the socket portion 232 is inserted into the opening 243 in the cap portion 242, the taper 244 of the opening 243 provides an inwardly directed force which causes the upper section 239 to contract (causes the slots 241 to narrow). This tapered entrance 244 opens to form an annular lip 245 which is useful for engaging and retaining the annular lip 240 of the upper section 239 of the socket portion 232. The interior surface 246 of the cap portion has a constant diameter, therein permitting the inserted upper section 239 of the socket portion 232 to slide and rotate relative to the cap portion 242.

The exterior surface of the cap portion 242 comprises a threading 247 which is designed to engage the threading 213 in the upper portion of the corresponding hole 210 or 212. In addition, the cap portion 242 comprises an axial hole 248 through which a surgeon may insert a screw driving tool to access the head of the screw which is positioned in the interior volume 234 of the socket portion 232.

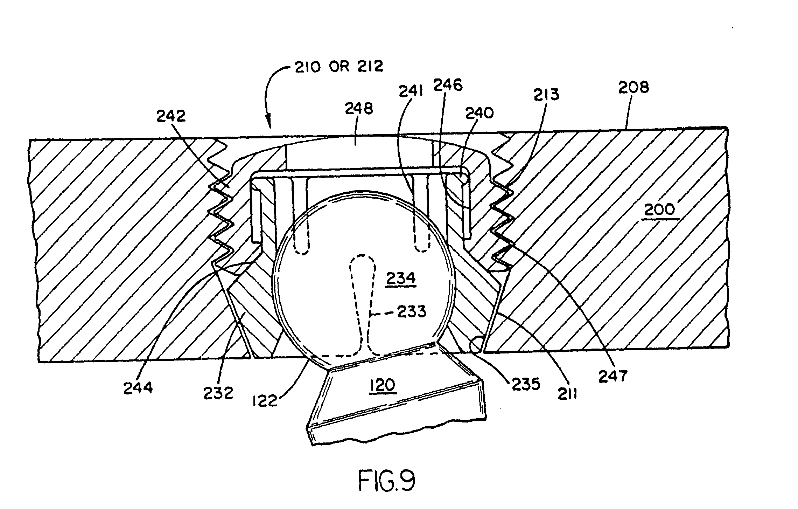

More particularly, with respect to the disposition of the head 122 of the screw 120 in the socket portion 232, and with reference to Figure 9, a fully assembled plate, coupling element, and screw is shown in a side cross-section view. The top 236 of the socket portion 232 is inserted into the opening in the cap portion 242 until the annular lip 240 of the socket 232 seats into the cap 242. The screw 120 is loosely held within the socket 232, which is, in turn, loosely retained within the cap 242.

With respect to the imploantation of the device, as with the first two embodiments, the plate 200 is positioned against the vertebral bones which are to be immobilized. A drill is used to pre-form holes into which the bone screws 120 are to be inserted (at the desired angulation). The screw 120 is then inserted and driven downward into the bone by use of the appropriate screw driving tool. As the screw 120 is driven deeper into the bone, the coupling element mounted to the head of the screw begins to enter the hole 210 or 212 of the plate 200. As the coupling element enters the hole 210 or 212, the tapered exterior surface 235 of the socket portion 232 seats against the tapered bottom 211 of the hole 210 or 212. Continued driving of the screw into the bone causes the slot 233 in the bottom of the socket portion 232 to narrow, thus causing the interior volume 234 of the socket portion 232 to crush against the head 122 of the screw 120.

The cap portion 242 of the coupling element may then be threadably advanced into the top section of the hole. As it is advanced. the upper annular lip 240 of the socket portion 232 slides upwardly along the inner surface 246 of the cap 242 until the bottom tapered opening 244 contacts the widened taper position of the socket portion 232. Continued advancement of the cap portion 242 provides further advancement of the socket portion 232 into the hole 210 or 212, thereby increased locking pressure within the interior volume 234 against the head 222.

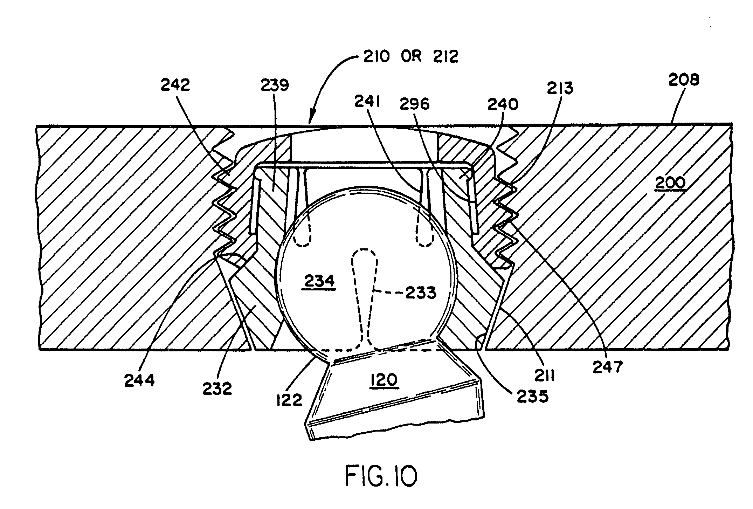

Referring to Figure 10, a variation of this two-piece coupling element device is shown in a similar cross-section view. In this embodiment, the inner surface 296 of the cap portion 242 is tapered inwardly in the vertical direction so that the advancement of the cap portion 242 along the threading 213 of the hole 210 or 212 causes the annular lip 240 to be compressed inwardly. This causes the slots 241 of the upper section 239 of the socket portion 232 to narrow. This may be utilized to further clamp the interior volume 234 against the head 122 of the screw 120. Once screwed into the plate 200, and locked down, the cap portion 242 of the coupling element and the top surface of the plate 208 present a flush external surface.

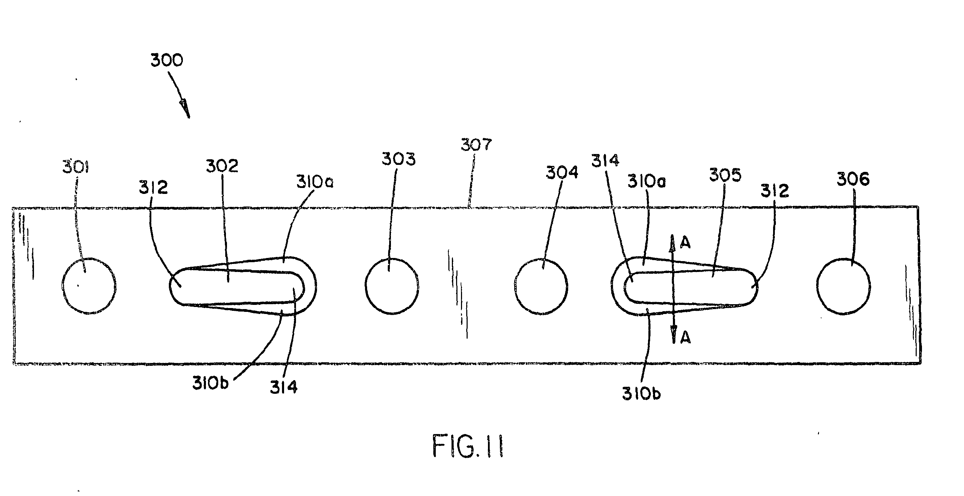

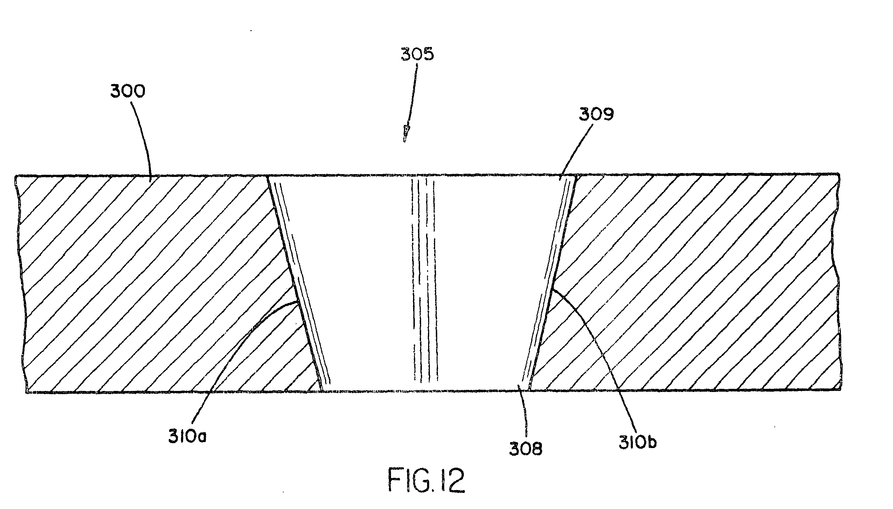

Referring now to Figure 11, a top view of the plate of the first embodiment of the present invention is shown wherein axis A-A is provided as a reference to a subsequent cross-section view provided in Figure 12. The plate 300 comprises an longitudinally extended, generally rectangular, thin metal plate, having a plurality of holes 301-306 defined therein. The holes 301-306 are divided into two groups of three, sequentially and symmetrically disposed on either sides of the longitudinal middle 307 of the plate. Holes 301, 303, 304, and 306 are provided for holding screws which provide additional securing strength once the initial affixation of the assembly, and the compression associated therewith, has been carried out. These holes and the corresponding screws are preferably of one of the types described above.

Holes 302 and 305 are disposed on either side of the middle 307 of the plate 300, and are preferably equidistant therefrom. The holes 302 and 305 are oriented in opposite sense with respect to the middle 307 of the plate 300, such that remote ends 312 thereof are farther from the middle 307 than the proximal ends 314. These holes 302 and 305 are elongated in the longitudinal direction of the plate 300. having tapered sidewalls 310a,310b.

More specifically with respect to the sidewall morphology, and referring also to Figure 12, the sidewalls 310a and 310b of the elongate hole 305 (or 302) are tapered along axes which are perpendicular to both the plane of the plate 300, and to the elongated sense of the hole 305. The width of the hole 305 is narrower at the bottom 308 than at the top 309. The taper of the hole 305 is such that the separation of the side walls 310a,310b is wider at the proximal end 314 than at the remote end 312.

The coupling element used in conjunction with this type of elongate hole is equivalent to the smooth tapered unitary coupling element 132 shown in Figure 3a. The exterior surface of the element 132 mates with the surfaces morphology of the elongate holes 302,305. More specifically, the narrower bottom 142a of the coupling element 132 is slightly smaller than the distance between the sidewalls 310a,310b of the top 309 of the elongate hole, at the remote end 312, which is in turn considerably narrower than the top 136a of the coupling element. The tapered sidewalls 310a,310b of the plate 300 widen in the direction of the middle 307, such that the distance between the sidewalls 310a,310b of the elongate hole 305 at the proximal end 314, are only slightly smaller than the corresponding tapered axial diameter of the coupling element 132. At the proximal end 314, it is this slight difference in tapering which provides the compression lock of the coupling element 132 to both the screw head 120 and the plate 300.

The first step in the process of implanting this embodiment of the invention is to position the plate against the segments of the broken bone which require compression and immobilization, and to align the entry points for the screws. The next step is to pre-drill holes into the bones at the desired position along the elongate hole, and angle with respect to the plate, into which the screws will be inserted. With the plate in place. one screw 120 and corresponding coupling element 132 are inserted through respective elongate holes 302,307 into the bone segments. In this embodiment, the screws may be inserted at any point along the length of their corresponding holes 302.305.

Once the screw has been inserted into the bone, at the desired angle, the coupling element 132, via its rotationally free mating of the element 132 to the head 122 of the screw, is realigned so that it may be seated against the tapered surfaces 310a,310b, in the elongated hole 302,305. If the screw is inserted at a point which is not directly in the proximal end 314, for example at the extreme other end, the remote end 314, the seating of the coupling element 132 is not as deep as it could be. Continued rotation of the screw 120, therefore, causes the coupling element 132 to slide deeper into the hole, which correspondingly requires that the screw 120 and bone move relative to the plate 300. The coupling element 132 continues to slide down the ever widening tapered walls 310a,310b of the hole, until it reaches the proximal end 314. At this proximal end 314, as the sidewalls 310a,310b remain slightly narrower than the coupling element 132, continued driving of the screw into the bone causes the slot 149 to close, therein crush locking the coupling element 132 to the screw head 122, and further therein compression locking the coupling element to the plate.

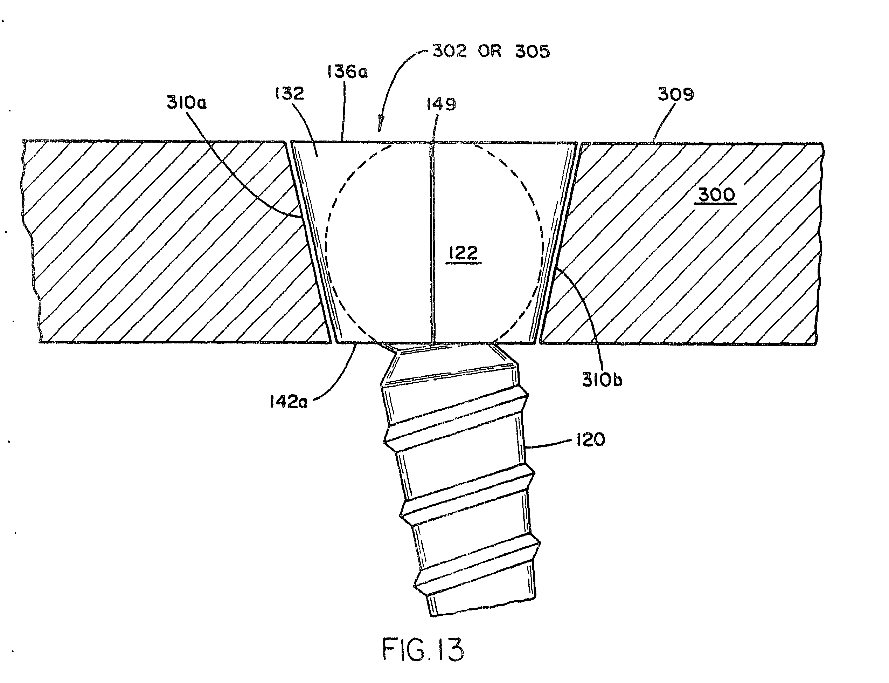

Referring now to Figure 13, a cross-section view of the fully seated and locked screw 120 and coupling element 132 is shown. As stated above, and shown herein, the side walls 310a,310b of the elongate hole 302,305 are slightly narrowed so that the slot 149 is narrowed, therein crush locking the head 122 to the element 132, and compression locked to the plate 300.

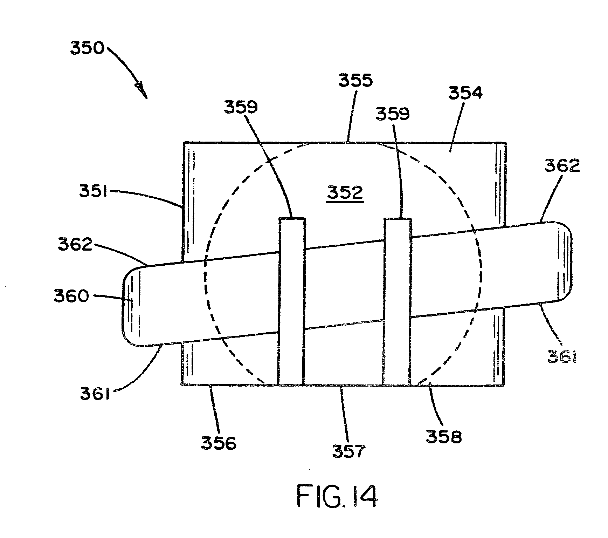

Referring now to Figure 14, the coupling element 350 of a further embodiment of the dynamic compression plate variation of the present invention is shown in a side view. As in the coupling element 132 of the first embodiment, the body 351 of the element comprises an interior volume 352, into which the semi-spherical head 122 of a screw 120, such as is shown in Figure 2, may be polyaxially disposed. The top 354 of the coupling element comprises a hole 355 through which the surgeon may access the screwdriving hole in the head 122 of the screw 120. The bottom 356 of the coupling element 350 comprises a hole 357 through which the head of the screw may be inserted. The hole 357 has a diameter which is less than the major diameter of the screw head 122, so that the screw may be held therein. The mouth of the bottom hole 357, therefore, includes an annular lip 358 which prevents the ball head from escaping the inner volume 352. In order to provide a means by which the screw head 122 may be inserted into the coupling element 350, the bottom of the element 350 may include slots 359 which expand under the application of a radial force outward. therein expanding the mouth of the hole 357 to receive a larger diameter object therethrough. The slots 359 further permit the bottom 356 of the coupling element 350 to be compressed inward, locking the element to the head 122 of the screw, upon the application of a radially inward force. (It shall be understood that the slots may alternatively descend from the top 354 of the element 350. In such an embodiment, the screw 120 would be inserted axially through the coupling element 350 from the top, and the top opening 355 would expand to receive the ball head 122 of the screw. This embodiment would lock the screw 120 in interior volume 352 of the coupling element by narrowing the slots in the same manner as the embodiment shown in Figure 14.)

The coupling element further comprises an annular flange portion 360 which extends outward from the cylindrical body. The flange 360 defines a circular ring which is offset slightly with respect to the axis of the coupling element body 351. The flange 360 has parallel upper and lower surfaces 362 and 361, respectively, and is ideally suited for travelling within a pair of parallel sloped channels as are described hereinbelow with respect to the corresponding plate of this embodiment.

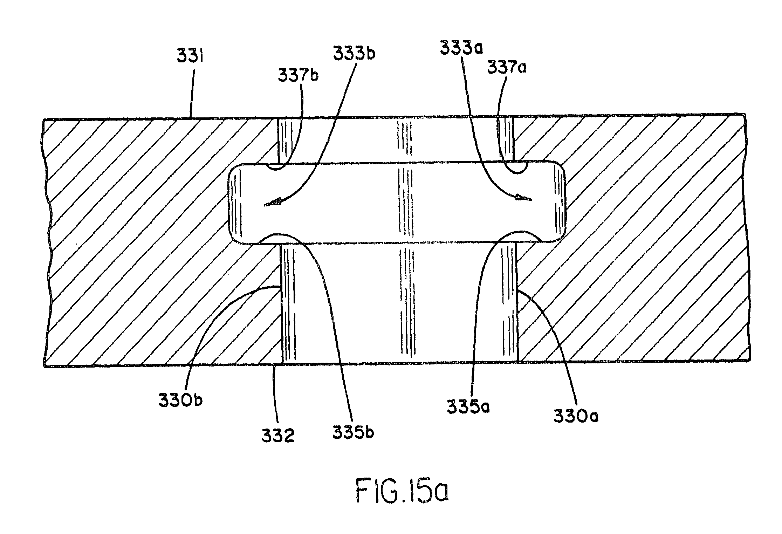

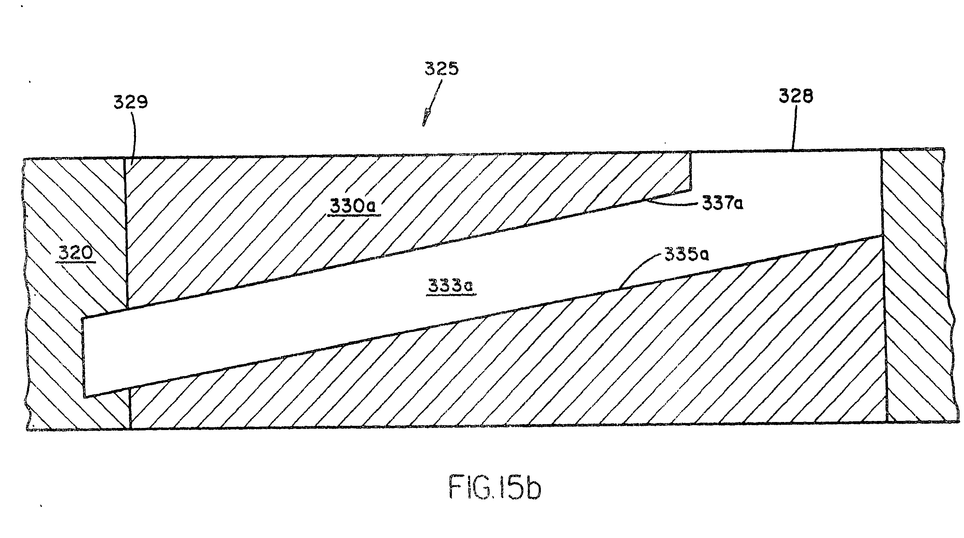

Referring now to Figures 15a and 15b, the plate 320 (and an elongate dynamic compression hole) of this embodiment of the present invention is shown in two mutually perpendicular cross-sectional views. More particularly, Figure 15a shows the a cross-section view of the hole which is transverse to the axis of the hole, and illustrates that the parallel sidewalls 330a,330b of the hole comprise, respective, recessed channels 333a,333b. The channels 333a,333b include defining lower ledges 335a,335b and upper ledge 337a,337b. Figure 15b shows an axial cross-section view of the hole, and illustrates that the channels 333a,333b are sloped downward along the sidewalls 330a,330b. At remote end 328 the channels 333a,333b are open so that the lower ledge 335a.335b may receive thereon the lower side 361 of the offset annular flange 360 of coupling element 350. The offset angulation of the flange 360 is ideally set to match the angulation of the sloped channels 333.

The initial steps of the implantation of the second embodiment of the present invention, including the preparation of the bone and the pre-drill holes, are similar to the preparatory steps associated with the first embodiment. Once positioned properly, the screws 120 are inserted in the bone through their respective elongate hole until the corresponding coupling elements 350 have properly seated in the remote ends 328 of the elongate holes, with the lower surface 361 of the flanges 360 of each element 350 disposed on the lower ledges 335a,335b of the channels 333a,333b. From this point further, continued driving of the screw into the bone causes the flange 360 of the coupling element 350 to slide down the sloped channels 333a,333b until the head has reached the deepest point at the proximal end 329. This translation of the coupling element down the channels 333a,333b. necessarily causes the bone into which the screw has been inserted to move relative to the fixed plate. As the process is carried out in opposing directions by the oppositely oriented elongate holes, displaced bone segments may be brought together and held fixed in contact.

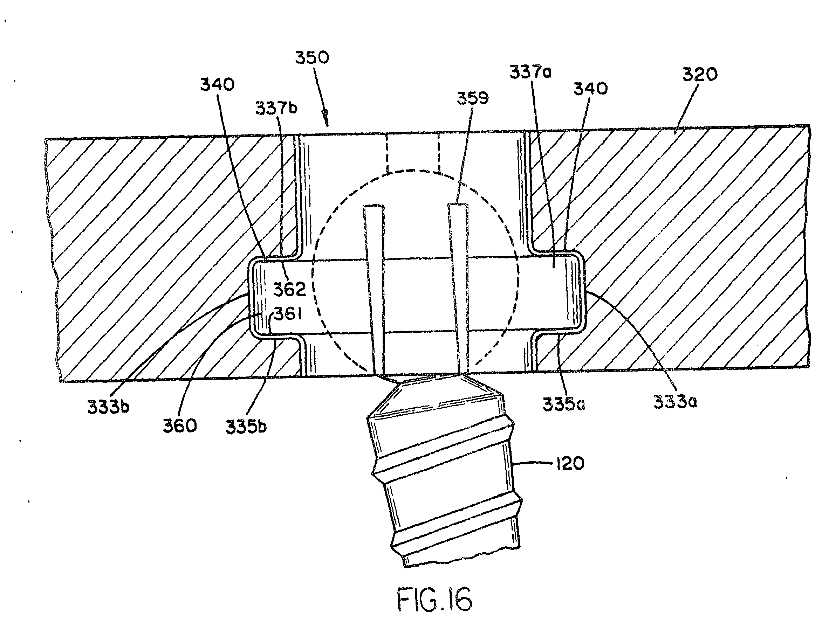

In a preferred variation, the channels 333a,333b narrow slightly along the longitudinal direction of the elongate hole, in the direction perpendicular to the axial direction of the elongate hole. This slight narrowing provides an inwardly directed radial force which causes slots 359 to close, thus locking the polyaxial screw 120 to the coupling element 350, so that it does not rotate with respect thereto. In all variations, however, it is preferred that the upper ledges 337a,337b of each channel 333a,333b in each of the sidewalls 330a,330b provide contact surfaces against which the upper surface 352 of the offset annular flange 360 interfaces. This interface 340, as shown in Figure 16, which illustrates the screw 120 and coupling element 350 in their fully inserted position, prevents screw back-out. In some variations, however, the upper ledges 337a,337b are only provided at the proximal ends of the elongate holes so that the screws 120 and coupling elements 350 may be inserted at any point along the extent of the holes.

More specifically, Figure 16 is provided to illustrate the coupling element 350 fully inserted in the plate 320, with offset annular flange 360 (which appears parallel in this cross-section angle) fully nested between the upper and lower ledges 362,361, respectively, of the channels 333a,333b.

While there has been described and illustrated implantation devices for stabilizing and immobilizing bone segments by affixing a polyaxial locking screw plate thereto, it will be apparent to those skilled in the art that variations and modifications are possible without deviating from the broad spirit and principle of the present invention which shall be limited solely by the scope of the claims appended hereto.

## Claims

1. An orthopedic device comprising:
a screw having a semi-spherical head and a threaded shaft,
a coupling element having an axial hole extending therethrough for receiving therein the semi-spherical head of the screw such that the screw may be moved through a variety of angles relative to the axial hole, the coupling element further including at least one slot; and
a receiving member including a through hole having an interior wall surface, a portion of the interior wall surface of the through hole being shaped to receive the coupling element and the screw when the semi-spherical head of the screw is mounted within the coupling element,
wherein engagement of the coupling element and the interior wall surface selectively prevents the screw from moving relative to the axial hole, thereby locking the angle of the screw relative to the axial hole.

2. The orthopedic device as set forth in claim 1, wherein the semi-spherical head of the screw further includes a recess formed therein for receiving therein a screwdriving tool such that the screw may be advanced into a vertebral bone.

3. A polyaxial locking screw plate assembly the immobilization of bones, via fixation to surfaces thereof, comprising:
a plate having a plurality of through holes;
a corresponding plurality of coupling elements, each of said coupling elements being insertable into a corresponding one of said through holes; and
a plurality of bone screws, each of said bone screws having a semi-spherical head portion and a shaft, said shaft portion being insertable through the corresponding through hole and into the bone, and said semi-spherical head portion being rotationally freely mounted in the coupling element prior to insertion and such that the shaft of the bone screw and the coupling element may be inserted into a corresponding through hole and said shaft of the bone screw may be inserted into the bone at a selected angle within a predetermined range of angles including non-perpendicular angles relative to the plate and thereby locking said coupling element and said semi-spherical head to said plate at said selected angle.

4. The polyaxial locking screw plate assembly as set forth in claim 1, wherein said plate includes a first end and a second end, a first group of said through holes being disposed at said first end, and a second group of said through holes being disposed in said second end.

5. The polyaxial locking screw plate assembly as set forth in claim 2, wherein each of said groups of through holes in said first and second ends includes an interior surface threading, and wherein each of said coupling elements includes an exterior surface threading which is matable to said interior surface threading, whereby each of said coupling elements is insertable into said corresponding through hole via engagement of said interior and exterior surface threadings.

6. The polyaxial locking screw plate assembly as set forth in claim 1, wherein said coupling elements each comprise an interior semi-spherical volume, defined by a curved interior surface, which forms a receiving socket into which the semi-spherical head portion is inserted whereby the head portion of said screw is rotationally freely mounted in said coupling element.

7. the polyaxial locking screw plate assembly as set forth in claim 4, wherein said coupling element further comprises at least one slot which permit the interior semi-spherical volume to expand thereby facilitating the insertion of said semi-spherical head portion of said screw therein.

8. The polyaxial locking screw plate assembly as set forth in claim 5, wherein the corresponding through hole into which the coupling element is inserted is tapered inwardly, thereby causing, upon insertion of said coupling element into said corresponding through hole, the at least one slot to be narrow, causing the curved interior surface of the coupling element to lock the semi-spherical head portion of the screw at a definite insertion angle.

9. The polyaxial locking screw plate assembly as set forth in claim 1, wherein the head portion of each of said screws comprises a recess to which a screwdriving toll is matable for inserting said screw through the corresponding hole and into the bone.

10. The polyaxial locking screw plate assembly as set forth in claim 7, wherein each of said coupling elements comprises a top surface recess, through which said screwdriving tool may be inserted, and which top surface recess may be aligned with the recess in the head of the screw, by which screwdriver the screw may be inserted into the bone.

11. The polyaxial locking screw plate assembly as set forth in claim 1, wherein at least one of said plurality of through holes further comprise an annular recess formed in an upper sidewall portion of said hole; and wherein said assembly further comprises a snap-ring, said snap ring deflecting outward during the insertion of the coupling element to permit the coupling element into the through hole, and wsaid snap ring snapping back to an undeflected condition once the coupling element has fully seated in the through hole, thereby preventing the coupling element from backing out of the through hole.

12. A polyaxial locking screw plate assembly for the immobilization of bones, via fixation to surfaces thereof, comprising:
a plate having an upper portion and a lower portion, said upper and lower portions each having at least one through hole, each of said through holes having an upper threaded portion and a lower tapered portion;
a plurality of bone screws, each of said bone screws having a semi-spherical head portion and a shaft; and
a plurality of coupling elements, each comprising
a socket portion having a semi-spherical interior volume, upper and lower sections, and vertical slots formed on corresponding ones of said upper and lower sections, at least one of said slots rendering said interior volume expandable and contractable, said lower section having a tapered exterior surface for seating against the lower tapered portion of said corresponding through hole such that forceable advancement of socket portion into the tapered portion of the through hole causes the at least one of said slots to narrow whereby the semi-spherical interior volume contracts, and
a cap portion having an opening in a bottom thereof and an interior chamber extending upwardly therefrom for joining with, and slideably retaining therein, the upper section of said socket portion, and exterior threading which is mateable with the threading of the corresponding hole,
wherein said semi-spherical head portion is rotationally freely mounted within the semi-spherical interior volume of the socket portion of the coupling element prior to insertion, and
whereby the insertion of the shaft of the bone screw and the coupling element into the corresponding through hole. and the insertion of said shaft of the bone screw inserted the bone at a selected angle within a predetermined range of angles including non-perpendicular angles to the plate, causes the tapered exterior surface of the socket portion to seat and to be forceably advanced against the tapered portion of the through hole such that the semi-spherical head of the screw is locked within the contracted semi-spherical interior volume, and
whereby threaded advancement of the cap portion into said through hole provides additional forceable advancement of the socket portion against the tapered portion of the through hole.

13. The polyaxial locking screw plate assembly as set forth in claim 10, wherein said at least one through hole of each of said upper and lower portions of said plate comprises a pair of through holes.

14. The polyaxial locking screw plate assembly as set forth in claim 10, wherein the head portion of each of said screws comprises a recess to which a screwdriving tool is matable for inserting said screw through the corresponding hole and into the vertebral bone.

15. The polyaxial locking screw plate assembly as set forth in claim 10, wherein said socket portion comprises an opening in said upper section thereof, through which a screwdriving tool may be inserted, and which may be aligned with the recess in the head of the screw.

16. The polyaxial locking screw plate assembly as set forth in claim 13, wherein said cap portion comprises an opening in a top thereof, aligned with the opening in the upper section of the socket portion, through which said screwdriving tool may be inserted, and which top surface recess may be aligned with the recess in the head of the screw.

17. The polyaxial locking screw plate assembly as set forth in claim 10,
wherein said socket portion further comprises a substantially constant diameter upper section having an outwardly annular extending lip at an extreme end thereof,
wherein said opening in the bottom of the cap portion comprises an inwardly directed annular lip, and
wherein at least one of said vertical slots in the upper section of said socket portion renders the upper section thereof to be expandable and contractable such that the upper section of the socket portion may be forceably inserted into the opening in the bottom of the cap portion so that it may be retained in the interior chamber therein by mutual interference engagement of the inwardly directed annular lip of the cap portion and the outwardly extending annular lip of the socket portion.

18. The polyaxial locking screw plate assembly as set forth in claim 15, wherein the interior chamber of the cap portion comprises a tapered surface such that advancement thereof into the hole causes an inwardly directed force against the upper section of the socket portion, therein causing the at least one of said vertical slots in the upper section to narrow and causes the upper section to contract and further lock the head of the screw within the interior semi-spherical volume of the socket portion.

19. A dynamic compression polyaxial locking screw plate assembly for drawing together and immobilizing a plurality of bone segments, via fixation to surfaces thereof, comprising:
an elongate plate including first and second longitudinal portions, and a middle defined therebetween;
at least one elongate hole, extending through at least one of said first and second longitudinal portions, having a remote end and a proximal end thereof and lateral sidewalls connecting said ends, each of said sidewalls including a sloped surface conformation;
means in at least the other of said first and second longitudinal portions by which said plate is securable to a first one of said plurality of said bone segments;
at least one corresponding coupling element, having a semi-spherical interior volume, said coupling element being insertable into said at least one elongate hole, slidable along said sloped surface conformations, and lockable within said elongate hole; and
at least one corresponding bone screw having a semi-spherical head and a shaft, said shaft portion being insertable through the corresponding elongate hole and into a second one of said bone segments, and said semi-spherical head portion being rotationally freely mounted within the semi-spherical volume of the coupling element prior to the insertion and such that the shaft of the bone screw and the coupling element may be inserted into the corresponding elongate hole and said shaft of the bone screw may be inserted into said second bone segment at a selected angle within a predetermined range of angles including non-perpendicular angles relative to the plate and thereby locking said coupling element and said semi-spherical head to said plate at said selected angle as said semi-spherical head and said coupling element are advanced into said elongate hole and along said sloped surface conformations toward the middle of said plate.

20. The dynamic compression polyaxial locking screw plate assembly as set forth in claim 17, wherein each of said ends of said plate comprises at least one elongate hole.

21. The dynamic compression polyaxial locking screw plate assembly as set forth in claim 17, wherein said plate further comprises at least one round hole, and wherein said means comprises a holding screw, insertable through said at least one round hole which provides added fixation strength.

22. The dynamic compression polyaxial locking screw plate assembly as set forth in claim 17, wherein a lower portion of the at least one coupling element comprises a plurality of slots which permit the interior semi-spherical volume to expand to receive said head portion of said screw therein, and to contract to crush lock to said head.

23. The dynamic compression polyaxial locking screw plate assembly as set forth in claim 17. wherein the at least one coupling element further comprises a single slot which extends the entire length of said coupling element, which permits the interior semi-spherical volume to expand to receive said semi-spherical head, and whereby inward radial force causes the single slot to close and the coupling element to crush lock to the semi-spherical head of the screw and compression lock within the elongate hole.

24. The dynamic compression polyaxial locking screw plate assembly as set forth in claim 17, wherein the surface conformations of the sidewalls of the at least one elongate hole comprise an axially widening taper, and wherein said coupling element has a corresponding axial taper.

25. The dynamic compression polyaxial locking screw plate assembly as set forth in claim 17, wherein the surface conformations of the sidewalls of the at least one elongate hole comprise an axially sloped channel, and wherein said coupling element has a corresponding annular flange.

26. The dynamic compression polyaxial locking screw plate assembly as set forth in claim 17, wherein the semi-spherical head of said screw comprises a recess to which a screwdriving tool is mateable for inserting said screw into said bone segment, and wherein the coupling element includes an opening in a top surface thereof, through which said screwdriving tool may access said recess of said screw.

27. An orthopedic implantation device which locks a polyaxial screw to an implant structure through which it is inserted, comprising:
a coupling element, having a semi-spherical interior receiving volume, and at least one axial slot, said coupling element being insertable into a corresponding through hole in said implant structure;
a corresponding bone screw having a semi-spherical head and a shaft, said shaft portion being insertable through the corresponding through hole of said implant structure and into a bone, and said semi-spherical head portion being rotationally freely mounted within the semi-spherical interior volume of the coupling element prior to insertion and such that the shaft of the bone screw and the coupling element may be inserted into the corresponding through hole and said shaft of the bone screw may be inserted into said bone at a selected angle within a predetermined range of angles including non-perpendicular angles relative to the implant structure and thereby locking said coupling element and said semi-spherical head to said implant structure at said selected angle as said semi-spherical head and said coupling element are advanced into said through hole,
wherein full insertion of said coupling element into said corresponding through hole causes the at least one axial slot to narrow, therein locking said semi-spherical head within the semi-spherical interior volume of said coupling element and said coupling element to said implant structure.

28. The orthopedic implantation device as set forth in claim 25, wherein said at least one axial slot comprises a single axial slot which extends the entire length of said coupling element.

29. The orthopedic implantation device as set forth in claim 25, wherein said at least one axial slot comprises a plurality of slots which extend for a portion of said coupling element.

30. A screw and collar assembly for polyaxially locking said screw within a hole through an orthopaedic implant device, said hole having an axially tapered inner surface comprising:
a bone screw having a threaded shaft and a semi-spherical head;
a coupling element having an axially tapered exterior surface, said taper corresponding to the taper of the hole in said orthopaedic implant device;
said coupling element further including an interior semi-spherical volume for polyaxially retaining the semi-spherical head of said bone screw; and
said coupling element further including at least one axial slot formed therein, which at least one slot provides for an expansion and contraction of the interior semi-spherical volume upon the application of a radial force on the exterior surface thereof;
whereby said shaft of the bone screw may be inserted through said hole and into the bone at a selected angle within a predetermined range of angles including non-perpendicular angles relative to the tapered hole, such that upon complete insertion, the coupling element and the semi-spherical head therein are drawn into the hole, the tapered inner surface of which applies an inwardly directed radial force to the coupling element which is sufficient to lock the coupling element within the tapered hole, and to lock the semi-spherical head of the screw in the semi-spherical interior volume of the coupling element.
